# EUROPEAN PATENT SPECIFICATION

(11) **EP 1 895 915 B1**
(45) Date of publication and mention of the grant of the patent: **23.03.2016**
(21) Application number: 06772426.0
(22) Date of filing: 06.06.2006
(51) Int. Cl.: A61B 17/42, A61B 17/02, A61B 17/04, A61B 17/06, A61B 17/062, A61B 17/12, A61B 17/29

(54) **DEVICES FOR LIGATING UTERINE ARTERIES**
VORRICHTUNGEN ZUR VERBINDUNG VON GEBÄRMUTTERARTERIEN
DISPOSITIFS DE LIGATURE DES ARTERES UTERINES

(30) Priority: 06.06.2005 US 687734 P
(43) Date of publication of application: 12.03.2008
(73) Proprietor: AMS Research Corporation, Minnetonka, MN 55343 (US)
(72) Inventor: MUJWID, James, R., Minnetonka, MN 55343 (US); ARNAL, Kevin, R., Minnetonka, MN 55343 (US); LUND, Robert, E., Minnetonka, MN 55343 (US); PRICE, Brian, P., Minnetonka, MN 55343 (US); COX, James, E., Minnetonka, MN 55343 (US); GUENTHER, Kevin, V., Minnetonka, MN 55343 (US); OGDAHL, Jason, Westrum, Minnetonka, MN 55343 (US); BOUCHIER, Mark, S., Minnetonka, MN 55343 (US)
(74) Representative: Vossius & Partner Patentanwälte Rechtsanwälte mbB
(86) International application number: PCT/US2006/022105
(87) International publication number: WO 2006/133289

(56) References cited:
- WO-A-99/08606
- US-A- 5 336 229
- US-A- 5 700 273
- US-A- 5 797 927
- US-A- 5 908 428
- US-A1- 2003 233 104

## Description

### FIELD OF THE INVENTION

The present invention pertains to a surgical system particularly suited to effect uterine artery ligation in a minimally invasive manner to treat uterine fibroids or other conditions of the uterus.

### BACKGROUND OF THE INVENTION

Each year, many women undergo a surgical removal of the uterus (hysterectomy) due to the growth of muscular tumors of the uterus (leiomyoma or uterine fibroids) or for uterine cancer, adenomyosis, menorrhagia, uterine prolapse, and dysfunctional uterine bleeding (abnormal menstrual bleeding that has no discrete anatomic explanation such as a tumor or growth). The uterus has a pear-shaped, uterine body extending between a fundus extending right and left to junctions with the right and left Fallopian tubes and a uterine neck (cervix) that extends to the vagina. The uterus has a smooth muscle uterine wall (myometrium) with an interior uterine mucosa (endometrium) that lines a uterine cavity extending between the right and left Fallopian tubes and a cervical opening of the cervix to the interior of the vagina. The uterine body is supported within the pelvis by right and left ligamentous structures such that the uterine body (fundus) is bent (anteflexed) and tilted (anteverted) anteriorly over the bladder and separated from the sacrum by the bowel. The uterine cervix extends into a tissue cul-de-sac of the vagina such that a flexible, annular trough (formix) of the vagina surrounds and is integrally connected with the cervix.

The ligamentous structures each include a broad ligament (part of the peritoneum), a round ligament, an ovarian ligament, a uterosacral ligament, a cardinal ligament, and other tissue structures. The broad ligament is a broad fold of peritoneum extending over the uterus and from the lateral margins of the uterus to the wall of the pelvis; it is divided into the mesometrium, mesosalpinx, and mesovarium that extend on each lateral side of the uterus to the wall of the pelvis. The mesometrium is the portion of the broad ligament below the mesovarium, composed of the layers of peritoneum that separate to enclose the uterus. The mesovarium is a portion of the broad ligament of the uterus between the mesometrium and mesosalpinx, which is drawn out to enclose and hold the ovary in place. The uterosacral ligaments are parts of the thickening of the visceral pelvic fascia beside the cervix and vagina, passing posteriorly in the rectouterine fold to attach to the front of the sacrum. The cardinal ligaments are fibrous bands attached to the uterine cervix and to the vault of the lateral fornix of the vagina and are contiguous with the mesometrium sheathing the pelvic vessels including the right and left uterine arteries.

Oxygenated blood is provided to the normal uterine cells and the cells of uterine fibroids by a redundant arterial blood supply denoted in the bilateral left and right uterine arteries and the bilateral left and right ovarian arteries. The right and left uterine arteries branch from the internal iliac artery and cross over the ureter at the level of the internal ostium (os) of the cervix and each divides into ascending and descending limbs. The ascending limb runs tortuously upward, between the leaves of the broad ligament, and supplies horizontal anterior and posterior branches to the cervix and the corpus. The descending branch of the uterine artery turns inferiorly and supplies the vagina from the lateral aspect. The ascending and descending branches of the uterine arteries extend through and are enclosed within the cardinal ligaments.

Uterine leiomyomas, commonly known as fibroids or myomas, are well circumscribed, solid, benign tumors arising from the smooth muscle of the myometrium, and are composed of smooth muscle cells and extracellular matrix. Fibroids may occur in several locations within the uterine wall and are named subserosal, submucosal, or intramural depending on their location. Subserosal leiomyomas are located just under the uterine serosa and may be attached to the corpus by a narrow or a broad base. Intramural leiomyomas are found predominantly within the thick myometrium and may distort the uterine cavity or cause an irregular external uterine contour. Submucous leiomyomas are located within the myometrium proximate to the endometrium. Uterine leiomyomas or fibroids are clinically apparent in 20% to 25% of women during the reproductive years and cause symptoms necessitating treatment, typically surgical removal of the uterus.

The surgical removal of the uterus requires exposing it sufficiently, ligating and severing the arteries and Fallopian tubes, severing the broad ligament and other ligaments from the uterine body, and severing the cervix from the fornix. Thus, in addition to the loss of reproductive capability, a hysterectomy requires major invasive surgery that can involve excessive blood loss, prolonged convalescence, attendant pain and discomfort, and economic costs. Newer treatment methods have been developed or proposed for at least some of these diseases and conditions. Nevertheless, hysterectomy remains the treatment of choice to treat the conditions and diseases listed above while less drastic treatments continue to be explored.

In the case of uterine fibroids, intraluminal occlusion of the right and left uterine arteries has been demonstrated as efficacious in starving and killing fibroid cells in situ while leaving normal uterine cells intact. For example, uterine artery occlusion or embolization was demonstrated as effective in eliminating or lessening uterine fibroids in Ravina et al., "Arterial Embolization to Treat Uterine Myomata", Lancet, 1995; Vol. 344; pp. 671-692. In this technique, uterine arteries are accessed via a trans-vascular route from a common femoral artery disposing a delivery catheter within the left and right uterine arteries, and embolic coils are dispensed from the catheter into the uterine arteries to promote clotting and thereby occlude the arterial passageways. When the uterine arteries are occluded in this fashion (or in any other fashion), the normal uterine cells and the fibroid cells of fibroids within the fundus are deprived of one blood supply. However, as demonstrated by Ravina et al., the effect on the fibroid cells is greater than the effect on normal uterine cells. In most instances, the relatively faster growing fibroid cells require a higher volume of blood oxygen and die when starved of oxygen, leading to fibroid shrinkage and cessation or diminution of clinical symptoms. Various methods of intraluminal occlusion of the right and left uterine arteries near the upper and lower branches thereof are also set forth in U.S. Patent No. 6,602,251 and in U.S. Patent Application Publication No. 2004/0202694, for example.

Such catheter-based uterine artery embolization must be performed with fluoroscopic or other visualization equipment by an interventional radiologist trained in catheterization and embolization delivery techniques. Thus, other invasive or non-invasive or minimally invasive techniques have been proposed and clinically explored to access the bilateral uterine arteries trans-vaginally or in a laparoscopic approach from a skin incision to temporarily or permanently apply compressive force around and thereby close the uterine arteries.

For example, trans-vaginal uterine artery occlusion with sutures tied around the right and left uterine ligaments exposed via a cervical incision is described by Harmanli, MD et al., in "Trans-vaginal Uterine Artery Ligation in a Woman with Uterine Leiomyomas", Journal of Reproductive Medicine, May 2003, Vol. 48; pp. 384-386. In this minimally invasive approach through the vagina, the uterosacral and cardinal ligaments are exposed by an annular incision around of the cervix, and the sutures are tied around the right and left uterine arteries and the supporting ligaments to occlude the arteries. Preferably, sutures are tied around the right and left uterosacral ligaments supporting descending branches of the uterine arteries and at least a proximal portion of the right and left cardinal ligaments supporting ascending branches of the uterine arteries. Thus, it is necessary to surgically expose both ligaments in a trans- vaginal procedure that is customarily followed in the initial steps of performing a hysterectomy. Typically in a hysterectomy, the uterine arteries are first clamped, ligated, or cauterized to halt blood flow to the uterus before the supporting ligaments are severed along the uterine wall.

A wide variety of further uterine artery occlusion techniques have been proposed in U.S. Patent Nos. 6,254,601 and 6,546,933, for example, and in Burbank, et al., "Uterine Artery Occlusion by Embolization or Surgery for the Treatment of Fibroids: A Unifying Hypothesis-Transient Uterine Ischemia," The Journal of the American Association of Gynecologic Laparoscopists, November 2000, Vol. 7, No. 4 Supplement, pp. S3-S49. In particular, various methods and apparatus have been suggested to determine the location of the uterine arteries, effect either permanent of temporary cessation of blood flow through the uterine arteries to the uterus to starve uterine fibroids of sufficient oxygenated blood, and to verify the blood flow cessation.

Various additional tools and minimally invasive techniques are presented for applying permanent or resorbable sutures or occlusion devices including snares, clips, and clamps, about at least a portion of each cardinal ligament and uterine artery are disclosed in U.S. Patent Nos. 6,506,156, 6,550,482, 6,602,251, 6,635,065, and 6,638,286 and in U.S. Patent Application Publication Nos. 2002/0124853 and 2003/0120286. Typically, access to the uterine arteries and cardinal ligaments is obtained by instruments introduced trans-vaginally to make one or more incision through the fornix and to advance and affix the suture or occlusion device about a portion of each cardinal ligament and uterine artery tightly enough to diminish or halt oxygenated blood flow.

Furthermore, tools adapted to be trans-vaginally applied temporarily to occlude the right and left uterine arteries are disclosed in U.S. Patent Application Publication Nos. 2002/0124853, 2002/0165579, 2002/0183771, 2003/0120306, 2003/0191391, 2004/0097961, 2004/0092979, 2004/0097962, 2004/0153105, 2004/0158262, and 2005/0113852. In one approach, the distal end of the tool that is advanced into the vagina is advanced into the fornix alongside the cervix to stretch the fornix and compress the right and/or left uterine artery against itself or against the cervix. The tool distal end is not advanced through the wall of the fornix, and the approach is therefore characterized as non-invasive. The tool may include a blood flow sensor to assist in disposing the tool distal end against a uterine artery and to verify that blood flow is reduced or halted when the uterine artery is compressed. The tool is applied to the uterine artery or arteries for a sufficiently long time period to effect starvation and death of uterine fibroids and is then withdrawn, allowing oxygenated blood flow to be restored unless occlusion has taken place.

Despite these approaches, it would be desirable to provide improved surgical instruments, tools and/or occluding devices and procedures that can be safely, simply, and readily employed to effect temporary or permanent occlusion of the uterine arteries, particularly through a non-invasive or minimally invasive trans-vaginal approach. Since their gynecologist diagnoses the majority of the patients suffering from fibroids, there is an advantage to the patient to be treated with a procedure that can be performed by their gynecologists. Because of the perceived risk of patient loss to referral, many patients will go untreated or are recommended a hysterectomy to treat their symptoms. If a trans-vaginal approach that follows many of the steps of a hysterectomy, which most gynecologist can perform, were available for the treatment of fibroids, many more patients would benefit from the relief of their symptoms with a minimally invasive surgery that could be performed by their gynecologist.

US 5,908,428 describes stitching describes for use in suturing artificial heart valves to heart tissue during heart valve replacement surgery.

### SUMMARY

The preferred embodiments of the present invention incorporate a number of inventive features that address the above-described needs. The surgical system of the present invention is not necessarily limited to but particularly suited to effect uterine artery ligation in a minimally invasive manner to treat uterine fibroids or other conditions of the uterus by diminishing or blocking arterial blood flow to treat uterine diseases or disorders.

The system of the present invention may be employed to restrict or block uterine arterial blood flow to treat uterine fibroids or other conditions through a minimally invasive trans-vaginal approach placing two sutures around the uterine arteries and at least a portion of the ligaments surrounding the uterine arteries.
As such, the present invention comprises a system for ligating a uterine artery in a patient as defined by independent claim 1.

Preferred embodiments of the invention are set forth in the dependent claims. The system generally includes a suture transfer tool, suture transfer darts, and two sutures. The suture transfer tool includes an upper jaw that is pivotally joined to a lower jaw. The lower jaw of the tool includes a number of suture capturing pins. The suture transfer darts are configured to be tissue piercing and are positionable within the suture capturing pins. Each end of the suture is connected to a suture transfer dart. When operating the tool, i.e., pivoting and compressing the upper jaw towards the lower jaw, the suture transfer darts pierce through the tissue to either side of a patient's cardinal ligament, which contains the uterine artery, and simultaneously present an end of the suture to either side of the cardinal ligament. As such, the suture is in an open loop configuration about the cardinal ligament and can be tied tight to the ligament to occlude the uterine artery.

The upper jaw of the tool preferably includes a transfer hole opposite each suture capturing pin to receive and maintain the suture transfer darts upon activation of the tool. The transfer darts are preferably cut from the suture to enable tying of the loop. The system further includes a second suture that is secured to one of the same transfer darts as the first suture. Upon pivoting and compressing the upper jaw to the lower jaw, the piercing of the tissue presents the end of the second suture in a position intermediate the cardinal ligament and the uterosacral ligament allowing a loop to be created about the uterosacral ligament with the second suture that allows the uterosacral ligament to be tied off as well. The two sutures are preferably color-coded for easy identification. Each of the suture capturing pins preferably includes a slot that aligns the suture when the suture transfer dart is placed within the suture capturing pins.

A method for ligating a uterine artery, not forming part of the invention, may include the following steps: (1) simultaneously piercing the tissue to either side of a cardinal ligament, which contains the uterine artery; (2) while piercing, simultaneously delivering the ends of a suture through the tissue piercings so as to present one end of the suture to one side of the cardinal ligament and the other end of the suture to the opposite side of the ligament to thus present the suture in an open loop about the cardinal ligament; (3) pulling the suture tight to the cardinal ligament; (4) tying the ends of the suture tight about the cardinal ligament to occlude the uterine artery within.

The method may further include the steps of simultaneously delivering an end of a second suture through one of the tissue piercings such that the end of the second suture is intermediate the cardinal ligament and the uterosacral ligament allowing an open loop to be formed about the uterosacral ligament with the second suture; then pulling the second suture tight about the uterosacral ligament; and tying the ends of the second suture tight about the uterosacral ligament to occlude the arteries contained therein.

### BRIEF DESCRIPTION OF THE DRAWINGS

These and other advantages and features of the present invention will be more readily understood from the following detailed description of the preferred embodiments thereof, when considered in conjunction with the drawings, in which like reference numerals indicate identical structures throughout the several views, and wherein:
FIG. 1 is a drawing of a healthy uterus.
FIG. 2 is a drawing of a uterus having leiomyomas.
FIG. 3 is a perspective view of the tool of the present invention.
FIG. 4 is a detailed perspective view of the upper and lower jaws of the tool of the present invention.
FIG. 5 depicts a suture transfer dart of the present invention.
FIG. 6 provides a detailed view of the preferred embodiment of the suture transfer dart.
FIG. 7 is a surgical view of a uterus.
FIG. 8 is a surgical view of a uterus.
FIG. 9 is a surgical view of a uterus.
FIG. 10 is a surgical view of a uterus.
FIG. 11 is a drawing of a uterus indicating occlusion sites.
FIG. 12 is a drawing of a frontal view of a uterus indicating occlusion sites.
FIG. 13 depicts the tool of the present invention loaded with suture transfer darts.
FIG. 14 depicts the tool of the present invention having placed suture loops about the cardinal and uterosacral ligaments
FIG. 15 depicts the sutures, delivered by the tool of the present invention, tied off and occluding the cardinal and uterosacral ligaments.

### DETAILED DESCRIPTION

In the following detailed description, references are made to illustrative embodiments for carrying out the invention. The system of the present invention is not necessarily limited to but is particularly suited to effect uterine artery ligation in a minimally invasive manner to treat uterine fibroids or other conditions of the uterus. Thus, preferred systems are described for controlling uterine arterial blood flow by diminishing or blocking arterial blood flow to the uterus to treat diseases and disorders, e.g., uterine fibroids and uterine bleeding. However, it should be noted that the surgical system of the present invention may be additionally useful in a hysterectomy procedure.

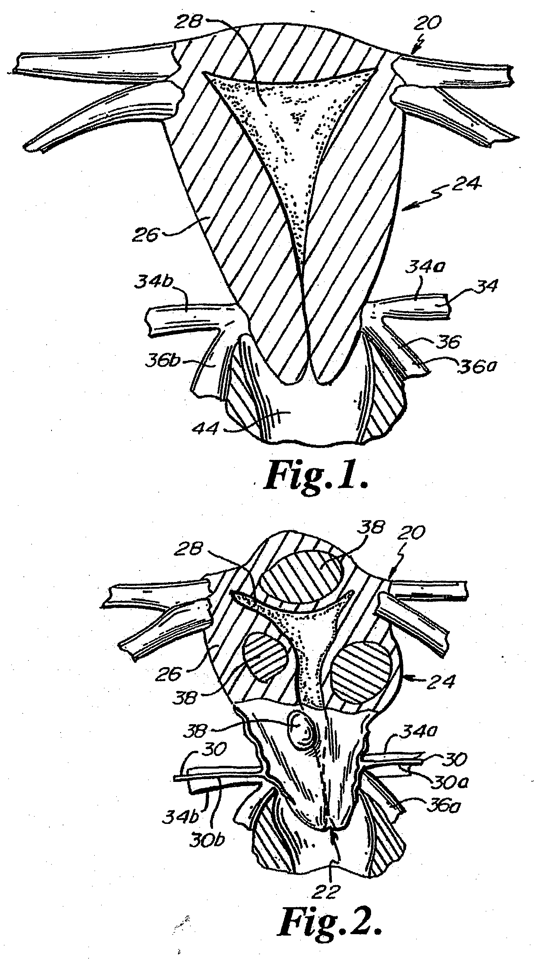

Reference is now made to FIG. 1, which illustrates the uterus 20 of a healthy female. The uterus 20 has a lower portion, which is known as the cervix 22, and an upper portion, known as the corpus 24. Among other structures, the uterus 20 includes uterine walls 26 and a uterine cavity 28. The uterus 20 accesses blood by a number of means. The most direct means is through the uterine arteries 30. Typically, a female has two uterine arteries 30; one artery generally on the left side 30a of the uterus 20 and one artery generally on the right side 30b of the uterus 20.

The uterus 20 is supported by means that include two cardinal ligaments 34, one on the left side 34a of the uterus 20 and one on the right side 34b of the uterus20. In addition, the uterus 20 is supported by two uterosacral ligaments 36, one on the left side 36a of the uterus 20 and one on the right side 36b of uterus 20. Typically, the uterine artery 30 is surrounded by the cardinal ligament 34 when the artery enters the uterus 20. Generally, the uterosacral ligaments 36 do not contain arteries. However some branches of a uterine artery may be in the vicinity of a uterosacral ligament36. It is also possible for the uterosacral ligament 36 and the cardinal ligament 34 to merge into a uterosacral cardinal complex which can attach to the uterus 20 in a merged manner. In addition, a uterosacral ligament 36 could include collateral source of blood that could flow to the uterus.

FIG.2 illustrates a uterus 20 with leiomyomas 38. Leiomyomas 38 are well known in the gynecological arts, and are generally described as benign smooth-muscle tumors. Leiomyomas 38 are also commonly known as fibroids. A leiomyoma 38 can attach to any portion of the uterus

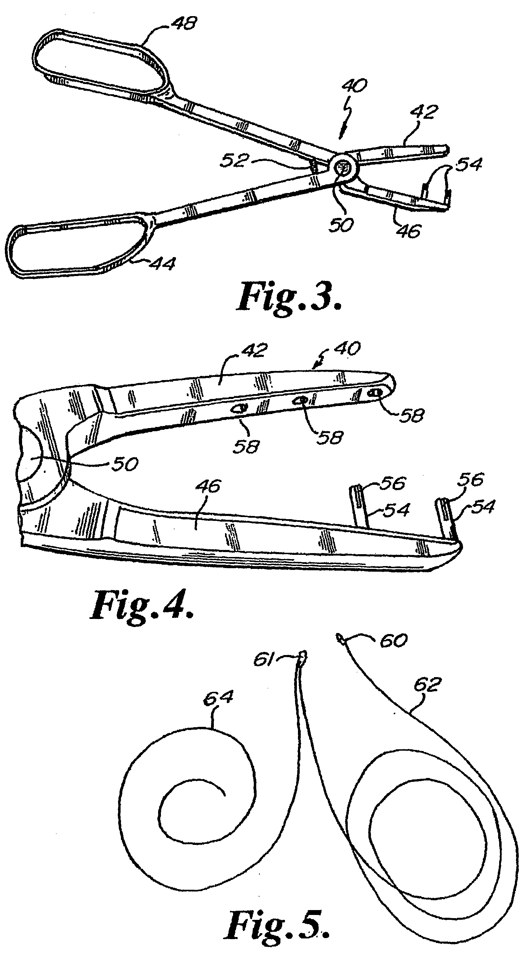

FIG.3 depicts a preferred embodiment of the tool 40 of the present invention. The tool 40 operates as a transvaginal uterine artery ligation (TVUAL) suture transfer instrument. The tool 40 is presented in a plier configuration having a pair of pivoted jaws. Specifically, the tool 40 includes an upper jaw 42 extending back to a first handle 44 and a lower jaw 46 extending back to a second handle 48.The jaws 42 and 46 are joined at pivot point 50. A spring 52 is provide intermediate the first handle 44 and the second handle 48 proximate the pivot point 50 to prevent over extension of the handles 44 and 48. A plurality of suture capturing pins 54 are provided on the lower jaw 46 of the tool 40.

FIG. 4 provides a detailed view of the upper and lower jaws, 42 and 46, respectively. As shown, each of the plurality of suture capturing pins 54 is provided with a hollowed center sized to accommodate a suture transfer dart (described further below). Additionally, each of the plurality of suture capturing pins 54 includes at least one slot 56 to accommodate an extending suture, i.e., a suture relief (described further below). Opposite the capturing pins 54 of the lower jaw 46 are a plurality of transfer holes 58 extending through the depth of the upper jaw 42. Each of transfer holes 58 is sized to accommodate reception of a suture transfer dart.

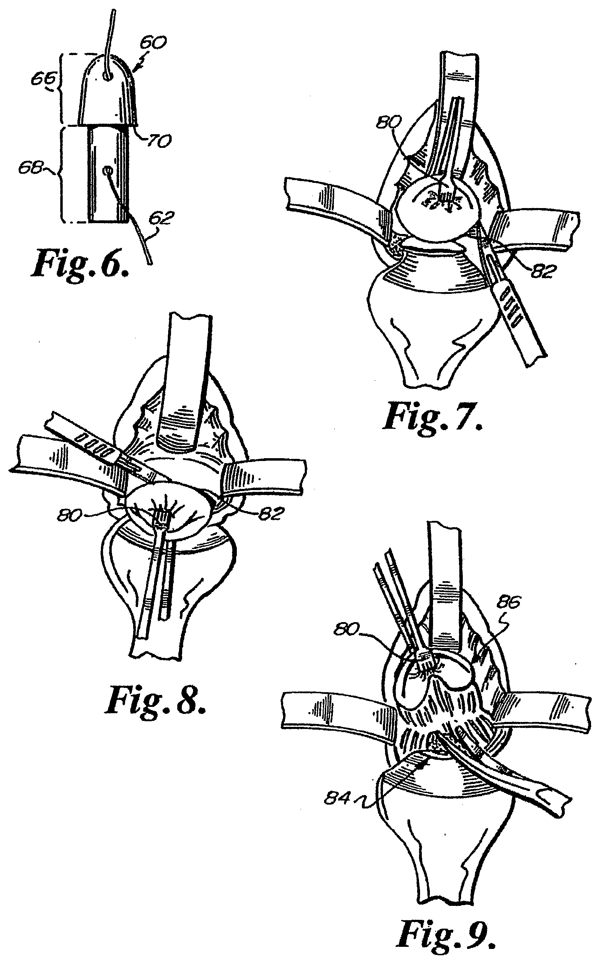

FIGS. 5 and 6 depict the preferred embodiment of the suture transfer darts 60 and 61, first suture 62 and second suture 64. As shown in FIG 6. each of suture transfer darts 60 and 61 has an upper portion 66 having a conical presentation, but may be any configuration suitable for penetrating tissue, and a lower portion 68 joined to the upper portion 66 via a chamfer 70. The lower portion 68 of each of the suture transfer darts 60 and 61 is pierced partially through in a manner perpendicular to its central axis to present an opening for reception of the suture(s), 62 and 64. In the preferred embodiment, suture 62 is secured between suture transfer dart 60 and suture transfer dart 61, while suture 64 is connected only at a first end to suture transfer dart 61 with its second end remaining free. The sutures 62 and 64 are preferably secured within the piercing of the suture transfer darts 60 and 61 with a drop of adhesive. In the preferred embodiment, suture 62 may be designated the cardinal ligament suture while suture 64 may be designated the uterosacral ligament suture; sutures 62 and 64 are preferably color-coded for easy differentiation between the two.

The tool 40 described above is preferably implemented through performance of the following procedure. With reference to FIGS. 7-9. General anesthesia is administered, the vaginal area 44 is prepared and draped, the patient is placed in a dorsal lithotomy position and the patient's bladder is drained. Then, the cervix can be exposed by a weighted speculum and vaginal retractors, and the cervix 22 is grasped with two tenacula 80. Without intending to be limiting, a Lahey tenacula may be used, as well as others known in the art. Thereafter, a circumferential cervical incision 82 is made outside the transformation zone of the uterus 20. FIG. 7 illustrates an incision 82 of the posterior portion of the cervix 22 and FIG. 8 illustrates an incision 82 of the anterior portion of the cervix 22. While the foregoing steps are described to create a circumferential incision 82 in the uterus 20, it is to be understood that, other methods currently know in the art can be used to prepare a patient and/or to enable vaginal access to the uterus 20. If only the cardinal ligament is to be ligated, the circumferential incision may be replaced with two smaller incisions, a superior and an inferior incision to the cervix, which allow both jaws 42 and 46 of the tool 40 to enter to the wound thereby enabling the final closure of the wound to be performed with less effort.

During the initial steps, it is also preferable to retract the bladder 42 away from the uterus 20. This will cause the ureters to be pulled away from the uterus 20 where they will be less likely to be impacted and/or damaged by the procedure.

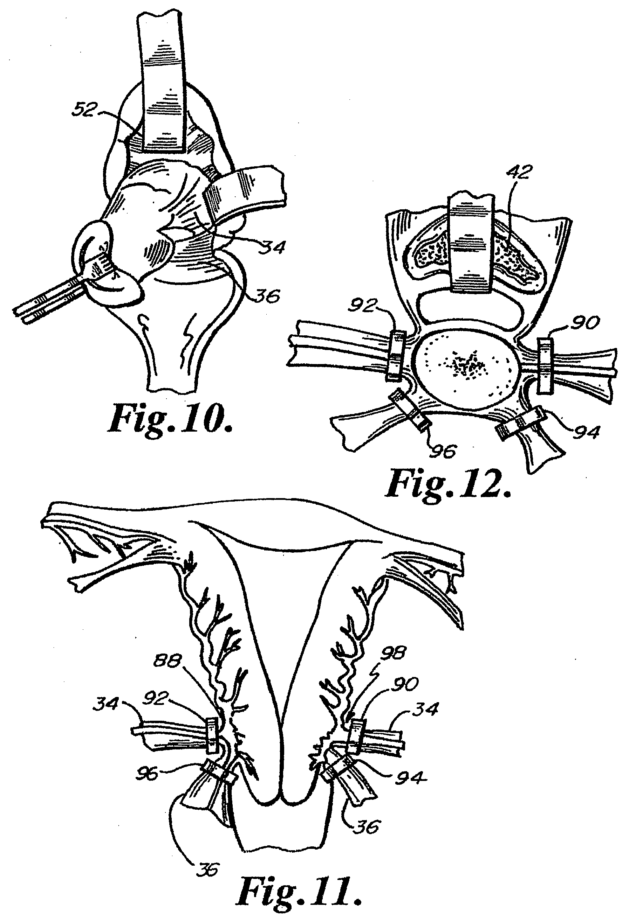

Next, the cul-de-sac 84 and avascular vesicouterine space 86 are entered. The cul-de-sac 84 is illustrated in FIG. 9 and the avascular vesicouterine space 86 is illustrated in FIG. 10. The order in which the cul-de-sac 84 and avascular vesicouterine space 86 are entered is discretionary with the physician. As known in the art, entering the cul-de-sac 84 and avascular vesicouterine space 86 can include separating the uterus 20 from the abdomen by incising or cutting through the vaginal mucosa, connective tissue and/or peritoneal layer. Excellent results have been achieved by sharply entering the cul-de-sac 84 and avascular vesicouterine space 86 by Mayo scissors. However, it is to be understood that other means currently known for entering the cul-de-sac 84 and avascular vesicouterine space 86 can be used.

Optionally, the weighted speculum can then be replaced with a longer, less obtrusive speculum to enable complete visualization of the cardinal and uterosacral ligaments.

Next, blood sources flowing through at least one uterine artery and/or one uterosacral ligament 36 to the uterus are occluded by ligation with sutures 62 and/or 64 delivered by tool 40. Generally, a uterine artery 30 can be occluded while located in the cardinal ligament 34 or after the artery is dissected away from the ligament. However, occluding the uterine artery 30 while it is located in the cardinal ligament 34 is preferable. Generally, the blood sources flowing through the uterosacral ligament 36 are collateral sources that might include a branch of the uterine artery. Occlusion of the blood sources through the uterosacral ligament 36 generally will occur while the blood source is located in the ligament.

It is to be noted that occlusion of the at least one uterosacral ligament 36 generally is optional because the ligament is not a main source of blood to the uterus 20. However, excellent results have been achieved by occluding the blood flowing through both the uterosacral ligaments 36 along with occluding the blood flowing through both uterine arteries 30. It is discretionary with the physician whether to occlude at least one uterosacral ligament 36. By way of example, and not intending to be limiting, a uterosacral ligament 36 might be occluded because it can be a collateral source of blood to the uterus 20. In addition, in some instances distinguishing between the uterosacral ligament 36 and the cardinal ligament 34 might be difficult due to the specific anatomy of the patient. Also, in some instances, the uterosacral ligament 36 and the cardinal ligament 34 merge near their insertion site with the uterus 20 at a uterosacral cardinal complex.

In addition, while occluding both uterine arteries 30 is recommended, there may be any number of reasons why occluding only one of the uterine arteries 30 could occur, while still obtaining desirable benefits. Similarly, there may be any number of reasons why occluding only one of the uterosacral ligaments 36 could occur, while still obtaining desirable benefits. By way of example and not intending to be limiting, a female patient could in rare cases possess only one uterosacral ligament 36 and/or only one cardinal ligament 34 due to genetic reasons or prior injury. In addition, there could be any number of reasons that the physician might decide to occlude only one of the uterosacral ligaments 36 and/or uterine arteries 30, which could include, but would not be limited to, injury, expediency and the like.

The order in which the at least one uterine artery 30 and optionally the blood sources in the uterosacral ligament 36 are occluded is discretionary with the physician. By way of example and not intending to be limiting, if all the uterine arteries 30 and the uterosacral ligaments 36 are going to be occluded, the physician might occlude the at least one uterosacral ligament 36 first because it is closer to the cervical incision 82 than the uterine artery 30. Alternatively, the physician might want to occlude the at least one uterine artery 30 first because the uterine arteries 30 are a main source of blood to the uterus 20. Thereafter, the physician might take a measurement of the blood flow to the uterus 20 before deciding whether to also occlude the at least one uterosacral ligament 36. Any number of factors could influence the physician's choice relating to the order of occlusion. Also, in the preferred case where both of the uterine arteries 30 are occluded the order of occlusion in relation to left and right arteries is discretionary. Similarly, where both uterosacral ligaments 36 are occluded, the order of occlusion in relation to left and right ligaments is discretionary.

Reference is now made to FIGS. 11 and 12, which indicate preferred occlusion points. The occlusion points are the general locations of the part of the ligament and/or artery that is manipulated to create the occlusion of the blood flow. The manipulation is performed by tool 40 and the sutures 62 and 64 presented thereby. In the preferred embodiment, as illustrated in these figures, the occlusion points are located on the ligament and/or artery immediately lateral to the isthmic 88 portion of the uterus 20. The occlusion points could also be referred to as being immediately lateral to the insertion site of the ligament to the uterus 20. Now specifically referring to the Figures, occlusion point 90 is on the left cardinal ligament 34, occlusion point 92 is on the right cardinal ligament 34, occlusion point 94 is on the left uterosacral ligament 36 and occlusion point 96 is on the right uterosacral ligament 36. However, if necessary and/or desired the occlusion point can be located at other places along the at least one cardinal ligament 34 without departing from the intent and scope of the current invention. When the occlusion point is not located immediately lateral to the isthmic 88 portion it is important to avoid impacting or damaging the ureter, which is located in the vicinity.

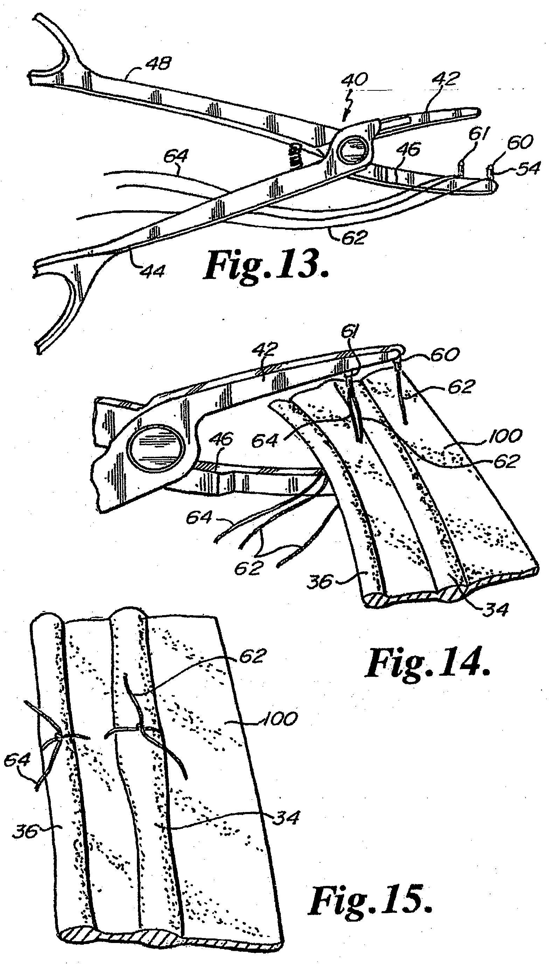

The occlusion points identified above are preferably located within a patient via palpation, however, a light source attached to the tool 40 or a retractor and/or a Doppler device could also be used to locate the occlusion points. Now referring to FIGS 13 and 14, tool 40 is prepared for use. Suture transfer darts 60 and 61, provided with sutures 62 and 64 already attached, are loaded into suture capturing pins 54, one dart per pin, with the sutures 62 and 64 extending through slot 56. The remaining length of the sutures 62 and 64 is drawn back proximate the handles 44 and 48. The handles are then actuated by the physician to press upper jaw 42 and lower jaw 46 of tool 40 towards each other with the pressure being exerted upon the jaws 42 and 46 causing the suture transfer darts 60 and 61 to penetrate the tissue 100, enter into the transfer holes 58 and be retained therein enabling the sutures 62 and 64 to be drawn upwards upon release of the jaws 42 and 46. As can be noted in FIG 12, the suture capturing pins 54 are spaced to present suture transfer dart 60 proximate the cardinal ligament 34 and the suture transfer dart 61 intermediate the cardinal ligament 34 and the uterosacral ligament 36. As such, suture 62 is presented in a loop about the cardinal ligament 34 and suture 64 is positioned to create a loop about the uterosacral ligament 36.

Tool 40 is then withdrawn from the occlusion location drawing the sutures 62 and 64 about the cardinal ligament 34 and uterosacral ligaments. Sutures 62 and 64 may then be cut from the suture transfer darts 60 and 61, which remain in the upper jaw 42 of the tool 40, with a scissors. The sutures 62 and 64 are then pulled tight and tied off to occlude the cardinal ligament and the uterosacral ligament, see FIG. 15. The sutures 62 and 64 may provide for permanent occlusion or may be of a bioresorbable material enabling temporary closure of the uterine artery 30. The result of occluding the ligaments is that blood flow to the uterus is reduced and fibroid ischemia occurs thereby reducing and/or eliminating the fibroid within the uterus. In an alternative embodiment only one of the sutures 62 or 64 is presented and delivered by tool 40 as desired.

The final step of the procedure is to approximate the cervical incision 82 by any appropriate technique known the art.

## Claims

1. A system for ligating a uterine artery (30) in a patient, comprising:
a suture transfer tool (40), wherein the suture transfer tool (40) includes an upper jaw (42) pivotally joined to a lower jaw (46), wherein said lower jaw (46) includes a plurality of suture capturing pins (54);
a plurality of suture transfer darts (60, 61), wherein said suture transfer darts are positionable within said plurality of suture capturing pins (54);
a first suture (62), wherein a first end of said first suture (62) is secured to a first of said plurality of suture transfer darts (60, 61) and wherein a second end of said first suture (62) is secured to a second of said plurality of suture transfer darts (60, 61), **characterized in that** each of said suture capturing pins (54) includes a slot (56) that aligns the first suture (62) attached to and extending from the suture transfer darts (60, 61) such that said first suture (62) extends through said slot; and
a second suture (64) having a first end secured to the second of said plurality of transfer darts (60, 61);
wherein upon pivoting and compressing said upper jaw (42) towards said lower jaw (46) of said tool (40), said plurality of suture transfer darts (60, 61) are configured to pierce through the tissue to either side of a cardinal ligament (34) containing the uterine artery (30) thereby presenting said first suture (62) in a loop about the uterine artery (30), wherein the loop is configured to be tied tight to occlude the uterine artery (30).

2. The system of claim 1, wherein said upper jaw (42) includes a plurality of transfer holes (58) opposite said plurality of suture capturing pins (54).

3. The system of claim 2, wherein said plurality of transfer holes (58) receive and maintain said plurality of suture transfer darts (60, 61).

4. The system of claim 3, wherein said suture transfer darts (60, 61) are cut from said suture to enable tying of said loop.

5. The system of claim 1, wherein the second of said plurality of suture transfer darts (60, 61) is positionable intermediate said cardinal ligament (34) and a uterosacral ligament (36) thereby presenting the second suture (64) in an approximate loop position about said uterosacral ligament (36), wherein the loop of the second suture (64) is configured to be tied tight to occlude the arteries within the uterosacral ligament (36).

6. The system of claim 1, wherein the first suture (62) and the second suture (64) are color coded.

## Patentansprüche

1. System zum Abbinden einer Gebärmutterarterie (30) in einer Patientin, das aufweist:
ein Nahtmaterialübertragungswerkzeug (40), wobei das Nahtmaterialübertragungswerkzeug (40) eine obere Backe (42) aufweist, die drehbar mit einer unteren Backe (46) verbunden ist, wobei die untere Backe (46) mehrere Nahtmaterialerfassungsstifte (54) aufweist;
mehrere Nahtmaterialübertragungspfeile (60, 61), wobei die Nahtmaterialübertragungspfeile in den mehreren Nahtmaterialerfassungsstiften (54) positionierbar sind;
ein erstes Nahtmaterial (62), wobei ein erstes Ende des ersten Nahtmaterials (62) an einem ersten der mehreren Nahtmaterialübertragungspfeile (60, 61) gesichert ist und wobei ein zweites Ende des ersten Nahtmaterials (62) an einem zweiten der mehreren Nahtmaterialübertragungspfeile (60, 61) gesichert ist, **dadurch gekennzeichnet, dass** jeder der Nahtmaterialerfassungsstifte (54) einen Schlitz (56) aufweist, der mit dem ersten Nahtmaterial (62) ausgerichtet ist, das an den Nahtmaterialübertragungspfeilen (60, 61) angebracht ist und sich von ihnen erstreckt, so dass sich das erste Nahtmaterial (62) durch den Schlitz erstreckt; und
ein zweites Nahtmaterial (64), von dem ein erstes Ende an dem zweiten der mehreren Übertragungspfeile (60, 61) gesichert ist;
wobei beim Drehen und Zusammendrücken der oberen Backe (42) zur unteren Backe (46) des Werkzeugs (40) die mehreren Nahtmaterialübertragungspfeile (60, 61) konfiguriert sind, durch das Gewebe zu einer Seite eines Ligamentum cardinale uteri (34) zu stechen, das die Gebärmutterarterie (30) enthält, wodurch das erste Nahtmaterial (62) in einer Schlinge um die Gebärmutterarterie (30) vorliegt, wobei die Schlinge konfiguriert ist, straff geschnürt zu werden, um die Gebärmutterarterie (30) zu verschließen.

2. System nach Anspruch 1, wobei die obere Backe (42) gegenüber den mehreren Nahtmaterialerfassungsstiften (54) mehrere Übertragungslöcher (58) aufweist.

3. System nach Anspruch 2, wobei die mehreren Übertragungslöcher (58) die mehreren Nahtmaterialübertragungspfeile (60, 61) aufnehmen und halten.

4. System nach Anspruch 3, wobei die Nahtmaterialübertragungspfeile (60, 61) vom Nahtmaterial abgeschnitten werden, um das Schnüren der Schlinge zu ermöglichen.

5. System nach Anspruch 1, wobei der zweite der mehreren Nahtmaterialübertragungspfeile (60, 61) zwischen dem Ligamentum cardinale uteri (34) und einem Ligamentum sacrouterinum (36) positionierbar ist, wodurch das zweite Nahtmaterial (64) in einer annähernden Schlingenposition um das Ligamentum sacrouterinum (36) vorliegt, wobei die Schlinge des zweiten Nahtmaterials (64) konfiguriert ist, straff geschnürt zu werden, um die Arterien im Ligamentum sacrouterinum (36) zu verschließen.

6. System nach Anspruch 1, wobei das erste Nahtmaterial (62) und das zweite Nahtmaterial (64) farbcodiert sind.

## Revendications

1. Système pour la ligature d'une artère utérine (30) d'un patient, comprenant :
un outil de transfert (40) de fil de suture, ledit outil de transfert (40) de fil de suture comportant une mâchoire supérieure (42) raccordée de manière pivotante à une mâchoire inférieure (46), ladite mâchoire inférieure (46) présentant une pluralité de broches de saisie (54) de fil de suture ;
une pluralité d'aiguillons de transfert (60, 61) de fil de suture, lesdits aiguillons de transfert de fil de suture étant positionnables à l'intérieur de la pluralité de broches de saisie (54) de fil de suture ;
un premier fil de suture (62), où une première extrémité dudit premier fil de suture (62) est fixé à un premier aiguillon de la pluralité d'aiguillons de transfert (60, 61) de fil de suture et où une deuxième extrémité dudit premier fil de suture (62) est fixée à un deuxième aiguillon de la pluralité d'aiguillons de transfert (60, 61) de fil de suture, **caractérisé en ce que** chaque broche de saisie (54) de fil de suture présente une fente (56) d'alignement du premier fil de suture (62) fixé aux aiguillons de transfert (60, 61) de fil de suture et s'étendant depuis ceux-ci, de telle manière que le premier fil de suture (62) s'étend dans la fente ; et
un deuxième fil de suture (64) présentant une première extrémité fixée au deuxième aiguillon de la pluralité d'aiguillons de transfert (60, 61) ;
où par pivotement et serrage de la mâchoire supérieure (42) vers la mâchoire inférieure (46) de l'outil (40), la pluralité d'aiguillons de transfert (60, 61) de fil de suture est prévue pour percer le tissu de part et d'autre du ligament de Mackenrodt (34) contenant l'artère utérine (30), le premier fil de suture (62) formant ainsi une boucle autour de l'artère utérine (30), ladite boucle étant destinée à être nouée de manière serrée pour occlure l'artère utérine (30).

2. Système selon la revendication 1, où la mâchoire supérieure (42) présente une pluralité d'orifices de transfert (58) opposée à la pluralité de broches de saisie (54) de fil de suture.

3. Système selon la revendication 2, où la pluralité d'orifices de transfert (58) reçoit et maintient la pluralité d'aiguillons de transfert (60, 61) de fil de suture.

4. Système selon la revendication 3, où les aiguillons de transfert (60, 61) de fil de suture sont coupés du fil de suture pour permettre de nouer la boucle.

5. Système selon la revendication 1, où le deuxième aiguillon de la pluralité d'aiguillons de transfert (60, 61) de fil de suture est positionnable entre le ligament de Mackenrodt (34) et un ligament utéro-sacré (36), le deuxième fil de suture (64) formant ainsi sensiblement une boucle autour du ligament utéro-sacré (36), la boucle du deuxième fil de suture (64) étant destinée à être nouée de manière serrée pour occlure les artères à l'intérieur du ligament utéro-sacré (36).

6. Système selon la revendication 1, où le premier fil de suture (62) et le deuxième fil de suture (64) sont pourvus d'un codage couleur.
